# EUROPEAN PATENT SPECIFICATION

(11) **EP 3 137 888 B1**
(45) Date of publication and mention of the grant of the patent: **02.06.2021**
(21) Application number: 15720550.1
(22) Date of filing: 24.04.2015
(51) Int. Cl.: G01N 27/02, G01F 23/26

(54) **SENSOR SYSTEM FOR MEASURING AN INTERFACE LEVEL IN A MULTI-PHASE FLUID COMPOSITION**
SENSORSYSTEM ZUR MESSUNG EINER FLUIDSCHNITTSTELLE IN EINEM MEHRPHASIGEN FLUID
SYSTÈME DE DÉTECTION D'UNE INTERFACE DANS UN FLUIDE MULTIPHASIQUE

(30) Priority: 02.05.2014 US 201461987853 P
(43) Date of publication of application: 08.03.2017
(73) Proprietor: BL Technologies, Inc., Minnetonka, MN 55341 (US)
(72) Inventor: SURMAN, Cheryl Margaret, Niskayuna, New York 12309 (US); DIERINGER, Jon Albert, Schenactady, New York 12309 (US); POTYRAILO, Radislav Alexandrovich, Niskayuna, New York 12309 (US)
(74) Representative: Marks & Clerk France
(86) International application number: PCT/US2015/027482
(87) International publication number: WO 2015/167955

(56) References cited:
- US-A1- 2003 169 032
- US-A1- 2013 285 677
- US-A1- 2014 090 451
- US-A1- 2014 090 454
- US-B1- 6 192 753

## Description

### RELATED APPLICATIONS

This application claims the benefit of US provisional application 61/987,853 filed on May 2, 2014.

### FIELD

The subject matter disclosed herein generally relates to sensors, and more particularly to level sensors to determine the interface level of a multi-phase fluid composition.

### BACKGROUND

Measurement of the composition of emulsions and the interface level of immiscible fluids is important in many applications. For example, it is important to characterize emulsions in oil field management. The measurement of the water and oil content of emulsions from individual oil wells may vary over the life of an oil field and may indicate the overall health of a field. In the case of injection wells, it is critical to control water quality to reduce hydrate formation and corrosion. Characterization of the composition of the oil and water mixture (e.g., measurement of the relative proportions of oil and water in the mixture) helps the operator improve well productivity and capacity. The information obtained is also useful to reduce back-pressure of wells, flowline size and complexity, and thermal insulation requirements.

Characterization of emulsions is also important in the operation of systems that contain fluids in a vessel (vessel systems) such as fluid processing systems. Vessel systems may include storage tanks, reactors, separators and desalters. Vessel systems are used in many industries and processes, such as the oil and gas, chemical, pharmaceutical, food processing industries, among others. For example, separation of water from raw oil is important to establishing production streams of oil and gas. Crude oil leaving the wellhead is both sour (contains hydrogen sulfide gas) and wet (contains water). The crude leaving the wellhead must be processed and treated to make it economically viable for storage, processing and export. One way of treating the raw oil is through the use of a separator. Most separators are driven by gravity and use the density differences between individual fluid phases of oil, water, gas, and solids to accomplish the separation. Identification of the interface levels of these layers is critical to the control of the separation process. Another fluid processing system where characterization of emulsions and measurement of the interface level is important is a desalter. Desalters are used in a refinery to control overhead corrosion downstream. In a desalter water and crude oil are mixed, inorganic salts are extracted into the water, and water is then separated and removed.

Finally, it is important to accurately characterize the water and salinity in the crude oil itself at various stages of the life of the product from a cost standpoint. Oil is a valuable commodity and underestimation of the water content in a typical tanker load can have significant cost consequences.

Wastewater management is another application where measurement and characterization of emulsion is important. Large quantities of oily wastewater are generated in the petroleum industry from both recovery and refining. A key factor in controlling the oil discharge concentrations in wastewater is improved instrumentation for monitoring the oil content of emulsions.

Many types of level and interface instruments have been contemplated over the years and a subset of those have been commercialized. Among those are gamma-ray sensors, guided wave sensors, magnetostrictive sensors, microwave sensors, ultrasonic sensors, single plate capacitance/admittance sensors, segmented capacitance sensors, inductive sensors, and computed tomography sensors. Each of the sensors has advantages and disadvantages. Some of the sensors are prohibitively expensive for many users. Some of the sensors may require a cooling jacket to perform at operating temperatures (above 125[deg.] C.). Some interface instruments require a clear interface to work, which can be problematic when working with diffuse emulsions. Some are susceptible to fouling. Other sensors do not have the ability to provide a profile of the tank, but rather monitor discreet points in the desalting process. Systems using electrodes are susceptible to the shorting of electrodes in high salinity applications and are susceptible to fouling. Finally, many of these systems are complex and difficult to implement.

Some existing sensor systems have used individual capacitive elements to measure fluid levels. A key limitation of those sensor systems is their inability to simultaneously quantify several components in the liquid. Capacitance methods have been used to measure dielectric constant of a liquid using specially designed electrodes for capacitance measurements. These designs are limited by the need for separate types of electrodes for capacitance measurements and for conductivity measurements. Inductor capacitor circuits also have been used to monitor the fluid level in a container using an electromagnetic resonator where change in capacitance was related to fluid level and fluid type. However, it has been the consensus of those of ordinary skill in the art that the filling of the resonator by a conducting liquid increased the uncertainties and noise in measurements by about one order of magnitude as compared to the values in a non-conducting fluid such as in air. However, these methods do not provide accurate measurements of concentrations of individual analytes at the limits of their minimum and maximum concentrations in the mixture.

With existing sensor systems, no one system is capable of delivering a combination of low cost, high sensitivity, favorable signal-to-noise ratio, high selectivity, high accuracy, and high data acquisition speeds. Additionally no existing system has been described as capable of accurately characterizing or quantifying fluid mixtures where one of the fluids is at a low concentration (i.e. at their minimum and maximum limits).

US 2014/090451 describes a resonant sensor system coupled to a sampling assembly.

### SUMMARY OF THE INVENTION

The disclosure provides a technical solution to the expense, reliability and accuracy problems of existing level sensor systems. An electrically resonant transducer (resonant transducer) provides a combination of low cost, high sensitivity, favorable signal-to-noise ratio, high selectivity, high accuracy, and high data acquisition speeds. The resonant transducer is incorporated in a robust sensor without the need for a clear interface. The solution also provides a sensor that is less susceptible to fouling, particularly in applications involving emulsions.

This disclosure describes, among other things, a sensor comprising a three-dimensional resonant transducer as claimed in claim 1. Preferably, the sampling cell comprises a tube or other structure adapted to locate a stationary or flowing fluid, for example oil or water.

The disclosure also relates to a sensor having a three-dimensional resonant transducer as claimed in claim 1 configured to determine a composition of an emulsion or other dispersion and a sensor system further including a sampling assembly and an impedance analyzer as claimed in claim 7.

In an embodiment, the disclosure relates to a system including a fluid processing system; a fluid sampling assembly; and a resonant sensor system coupled to the fluid sampling assembly.

In another embodiment, the disclosure relates to a method for measuring a level of a mixture of fluids in a vessel. The method includes the steps of detecting a signal from the sensor system at a plurality of locations in the vessel; converting each signal to values of the complex impedance spectrum for the plurality of locations; storing the values of the complex impedance spectrum and frequency values; and determining a fluid phase inversion point from the values of the complex impedance spectrum.

In another embodiment, the disclosure relates to a method for determining a composition of a mixture of oil and water in a vessel. The method includes the step of determining values of the complex impedance spectrum of the mixture of oil and water as a function of a height in the vessel with the resonant transducer. The method also includes the step of determining a fluid phase inversion point from the values of the complex impedance spectrum; applying an oil phase model to the values of the complex impedance spectrum and conductivity values above the fluid phase inversion point, and applying a water phase model to the values of the complex impedance spectrum below the fluid phase inversion point.

In another embodiment, the disclosure relates to a sensor comprising a three-dimensional resonant transducer configured to simultaneously determine concentration of a first and a second component of an emulsion.

### BRIEF DESCRIPTION OF THE FIGURES

Other features and advantages of the present disclosure will be apparent from the following more detailed description of the preferred embodiment, taken in conjunction with the accompanying drawings which illustrate, by way of example, the principles of certain aspects of the disclosure.
FIG. 1 is a schematic of a non-limiting embodiment of a resonant sensor system.
FIG. 2 is a non-limiting illustration of the operation of a resonant transducer.
FIG. 3 is an example of a measured complex impedance spectrum used for multivariate analysis.
FIG. 4 illustrates an embodiment of a two-dimensional resonant transducer.
FIG. 5 illustrates an embodiment of a three-dimensional resonant transducer, which is not part of the present invention.
FIG. 6 is a schematic electrical diagram of the equivalent circuit of a three-dimensional resonant transducer.
FIG. 7 is a chart illustrating the Rp response of a resonant transducer to varying mixtures of oil and water.
FIG. 8 is a chart illustrating the Cp response of a resonant transducer to varying mixtures of oil and water.
FIG. 9 is a partial cutaway side view of an embodiment of a resonant transducer assembly.
FIG. 10 is a schematic diagram of an embodiment of a fluid processing system.
FIG. 11 is a schematic diagram of an embodiment of a desalter.
FIG. 12 is a schematic diagram of an embodiment of a separator.
FIG. 13 is a chart illustrating the frequency (Fp) response of a three-dimensional resonant transducer to increasing concentrations of oil-in-water and water-in-oil emulsions.
FIG. 14 is a chart illustrating the frequency (Fp) response of a two-dimensional resonant transducer to increasing concentrations of oil-in-water and water-in-oil emulsions.
FIG. 15 is a flow chart of an embodiment of a method for determining the composition of an oil and water mixture as a function of height.
FIG. 16 is a chart illustrating data used to determine a fluid phase inversion point and conductivity.
FIG. 17 is a chart illustrating the results of an analysis of the experimental data of an embodiment of a resonant sensor system.
FIG. 18 is a chart illustrating test results of a resonant sensor system in a simulated desalter.
FIG. 19 is an embodiment of a display of a data report from a resonant sensor system.
FIG. 20 is a flowchart of an embodiment of a method for determining the level of a fluid in a vessel.
FIG. 21 is a block diagram of a non-limiting representative embodiment of a processor system for use in a resonant sensor system.
FIG. 22 illustrates another embodiment of a three-dimensional resonant transducer according to the present invention.

### DETAILED DESCRIPTION

As discussed in detail below, embodiments of the present invention provide low cost systems for reliably and accurately measuring the fluid level in a fluid processing vessel. A resonant sensor system provides effective and accurate measurement of the level of the transition or emulsion layer through the use of a resonant transducer such as an inductor-capacitor-resistor structure (LCR) multivariable resonant transducer and the application of multivariate data analysis applied to the signals from the transducer. The resonant sensor system also provides the ability to determine the composition of water and oil mixtures, oil and water mixtures and, where applicable, the emulsion layer.

The resonant transducer includes a resonant circuit and a pick up coil. The electrical response of the resonant transducer immersed in a fluid is translated into simultaneous changes to a number of parameters. These parameters may include the complex impedance response, resonance peak position, peak width, peak height and peak symmetry of the impedance response of the sensor antenna, magnitude of the real part of the impedance, resonant frequency of the imaginary part of the impedance, antiresonant frequency of the imaginary part of the impedance, zero-reactance frequency, phase angle, and magnitude of impedance, and others as described in the definition of the term sensor "spectral parameters." These spectral parameters may change depending upon the dielectric properties of the surrounding fluids. The typical configuration of a resonant transducer may include an LCR resonant circuit and an antenna. The resonant transducer may operate with a pickup coil connected to the detector reader (impedance analyzer) where the pickup coil provides excitation of the transducer and detection of the transducer response. The resonant transducer may also operate when the excitation of the transducer and detection transducer response is performed when the transducer is directly connected to the detector reader (impedance analyzer).

A resonant transducer offers a combination of high sensitivity, favorable signal-to-noise ratio, high selectivity, high accuracy, and high data acquisition speeds in a robust sensor without the need for optical transparency of the analyzed fluid and the measurement flow path. Instead of conventional impedance spectroscopy that scans across a wide frequency range (from a fraction of Hz to tens of MHz or GHz) a resonant transducer is used to acquire a spectrum rapidly and with high signal-to-noise across only a narrow frequency range. The sensing capability is enhanced by putting the sensing region between the electrodes that constitute a resonant circuit. As implemented in a fluid processing system such as a desalter or a separator, the resonant sensor system may include a sampling assembly and a resonant transducer coupled to the fluid sampling assembly. The resonant sensor system implements a method for measuring the level of a mixture of fluids in a vessel, and may also implement a method for determining the composition of a mixture of oil and water in a vessel. The resonant transducers are capable of accurately quantifying individual analytes at their minimum and maximum limits. The resonant sensor system is able to determine the composition of fluid mixtures even when one of the fluids is at a low concentration.

A nonlimiting examples of fluid processing systems include reactors, chemical reactors, biological reactors, storage vessels, containers, and others known in the art.

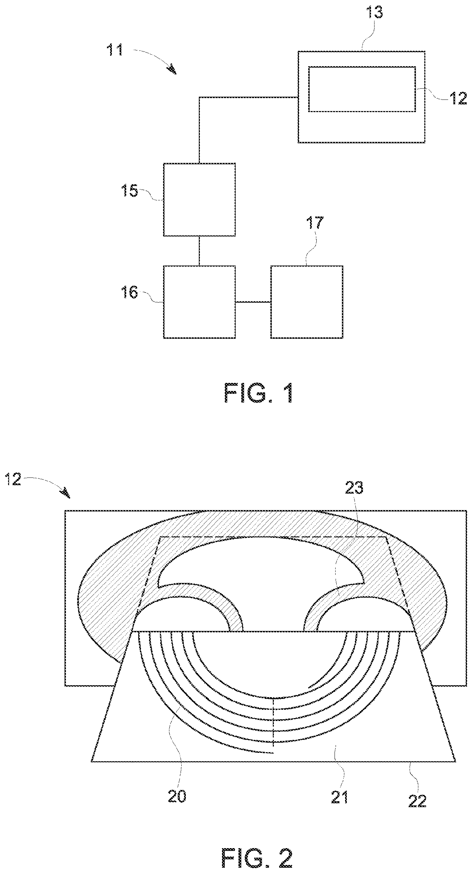

Illustrated in FIG. 1 is a schematic of an embodiment of a resonant sensor system 11. The resonant sensor system 11 includes a resonant transducer 12, a sampling assembly 13, and an impedance analyzer (analyzer 15). The analyzer 15 is coupled to a processor 16 such as a microcomputer. Data received from the analyzer 15 is processed using multivariate analysis, and the output may be provided through a user interface 17. Analyzer 15 may be an impedance analyzer that measures both amplitude and phase properties and correlates the changes in impedance to the physical parameters of interest. The analyzer 15 scans the frequencies over the range of interest (i.e., the resonant frequency range of the LCR circuit) and collects the impedance response from the resonant transducer 12.

As shown in FIG. 2, resonant transducer 12 includes an antenna 20 disposed on a substrate 22. The resonant transducer may be separated from the ambient environment with a dielectric layer 21. In some embodiments, the thickness of the dielectric layer 21 may range from 2 nm to 50 cm, more specifically from 5 nm to 20 cm; and even more specifically from 10 nm to 10 cm. In some applications the resonant transducer 12 may include a sensing film deposited onto the transducer. In response to environmental parameters an electromagnetic field 23 may be generated in the antenna 20 that extends out from the plane of the resonant transducer 12. The electromagnetic field 23 may be affected by the dielectric property of an ambient environment providing the opportunity for measurements of physical parameters. The resonant transducer 12 responds to changes in the complex permittivity of the environment. The real part of the complex permittivity of the fluid is referred to as a "dielectric constant". The imaginary part of the complex permittivity of the fluid is referred to as a "dielectric loss factor". The imaginary part of the complex permittivity of the fluid is directly proportional to conductivity of the fluid.

Measurements of fluids can be performed using a protecting layer that separates the conducting medium from the antenna 20. Response of the resonant transducer 12 to the composition of the fluids may involve changes in the dielectric and dimensional properties of the resonant transducer 12. These changes are related to the analyzed environment that interacts with the resonant transducer 12. The fluid-induced changes in the resonant transducer 12 affect the complex impedance of the antenna circuit through the changes in material resistance and capacitance between the antenna turns.

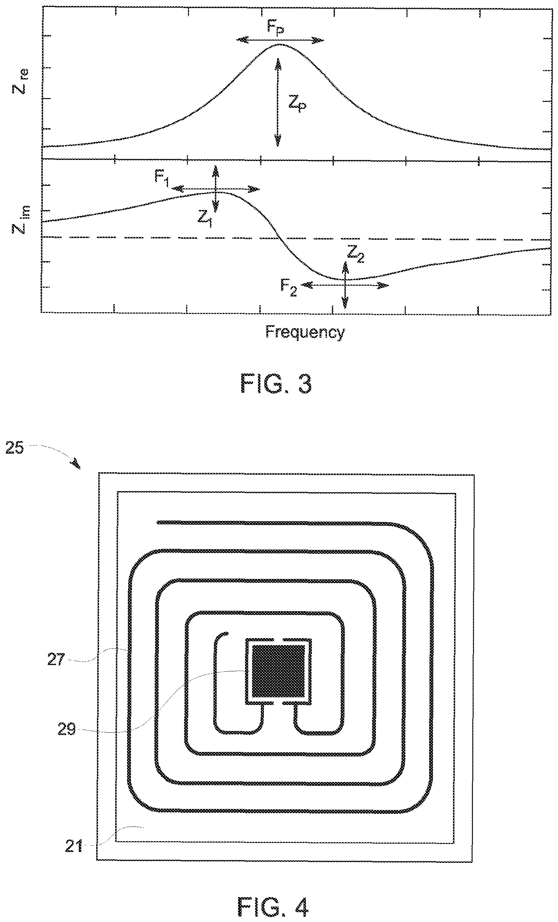

For selective fluid characterization using a resonant transducer 12, the complex impedance spectra of the sensor antenna 20 are measured as shown in FIG. 3. At least three data points of impedance spectra of the emulsion are measured. Better results may be achieved when at least five data points of the impedance spectra of the emulsion are measured. Non limiting examples of number of measured data points are 8, 16, 32, 64, 101, 128, 201, 256, 501, 512, 901, 1024, 2048 data points. Spectra may be measured as a real part of impedance spectra or an imaginary part of impedance spectra or both parts of impedance spectra. Non-limiting examples of LCR resonant circuit parameters include impedance spectrum, real part of the impedance spectrum, imaginary part of the impedance spectrum, both real and imaginary parts of the impedance spectrum, frequency of the maximum of the real part of the complex impedance (Fp), magnitude of the real part of the complex impedance (Zp), resonant frequency (F 1) and its magnitude (Z 1) of the imaginary part of the complex impedance, and anti-resonant frequency (F 2) and its magnitude (Z 2) of the imaginary part of the complex impedance.

Additional parameters may be extracted from the response of the equivalent circuit of the resonant transducer 12. Non-limiting examples of the resonant circuit parameters may include quality factor of resonance, zero-reactance frequency, phase angle, and magnitude of impedance of the resonance circuit response of the resonant transducer 12. Applied multivariate analysis reduces the dimensionality of the multi-variable response of the resonant transducer 12 to a single data point in multidimensional space for selective quantitation of different environmental parameters of interest. Non-limiting examples of multivariate analysis tools are canonical correlation analysis, regression analysis, nonlinear regression analysis, principal components analysis, discriminate function analysis, multidimensional scaling, linear discriminate analysis, logistic regression, and/or neural network analysis. By applying multivariate analysis of the full complex impedance spectra or the calculated spectral parameters, quantitation of analytes and their mixtures with interferences may be performed with a resonant transducer 12. Besides measurements of the complex impedance spectra parameters, it is possible to measure other spectral parameters related to the complex impedance spectra. Examples include, but are not limited to, S-parameters (scattering parameters) and Y-parameters (admittance parameters). Using multivariate analysis of data from the sensor, it is possible to achieve simultaneous quantitation of multiple parameters of interest with a single resonant transducer 12.

According to the invention, the resonant transducer 12 is characterized as three-dimensional.

Shown in FIG. 4 is a two-dimensional resonant transducer 25 which is not included in the scope of the claimed invention, having a transducer antenna 27. The two-dimensional resonant transducer 25 is a resonant circuit that includes an LCR circuit. In some embodiments, the two-dimensional resonant transducer 25 may be coated with a sensing film 21 applied onto the sensing region between the electrodes. The transducer antenna 27 may be in the form of coiled wire disposed in a plane. The two-dimensional resonant transducer 25 may be wired or wireless. In some embodiments, the two-dimensional resonant transducer 25 may also include an IC chip 29 coupled to transducer antenna 27. The IC chip 29 may store manufacturing, user, calibration and/or other data. The IC chip 29 is an integrated circuit device and it includes RF signal modulation circuitry that may be fabricated using a complementary metal-oxide semiconductor (CMOS) process and a nonvolatile memory. The RF signal modulation circuitry components may include a diode rectifier, a power supply voltage control, a modulator, a demodulator, a clock generator, and other components.

Sensing is performed via monitoring of the changes in the complex impedance spectrum of the two-dimensional resonant transducer 25 as probed by the electromagnetic field 23 generated in the transducer antenna 27. The electromagnetic field 23 generated in the transducer antenna 27 extends out from the plane of the two-dimensional resonant transducer 25 and is affected by the dielectric property of the ambient environment, providing the opportunity for measurements of physical, chemical, and biological parameters.

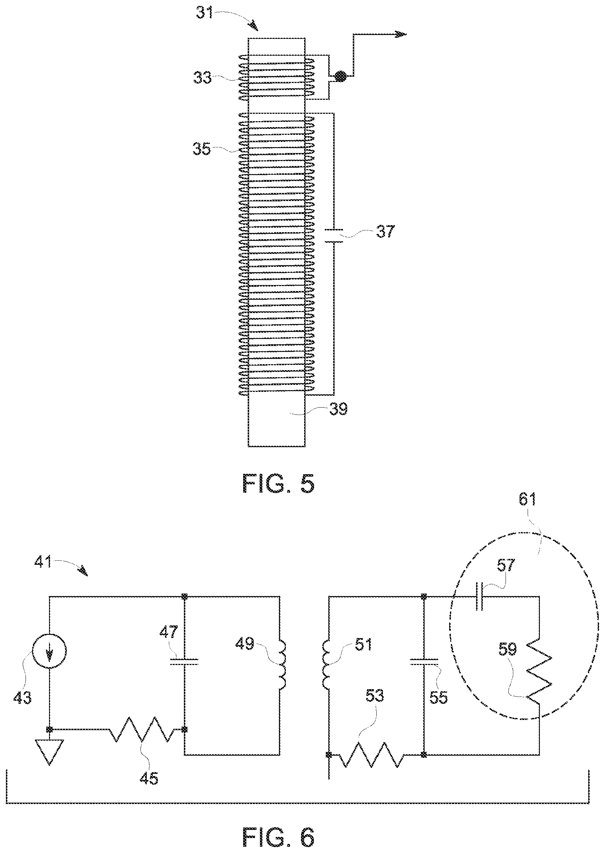

Shown in FIG. 5 is a three-dimensional resonant transducer 31 which is not included in the scope of the claimed invention. The three-dimensional resonant transducer 31 includes a top winding 33 and a bottom winding 35 coupled to a capacitor 37. The top winding 33 is wrapped around an upper portion of a sampling cell 39 and the bottom winding 35 is wrapped around a lower portion of the sampling cell 39. The sampling cell 39 may, for example, be made of a material resistant to fouling such as Polytetrafluoroethylene (PTFE), a synthetic fluoropolymer of tetrafluoroethylene.

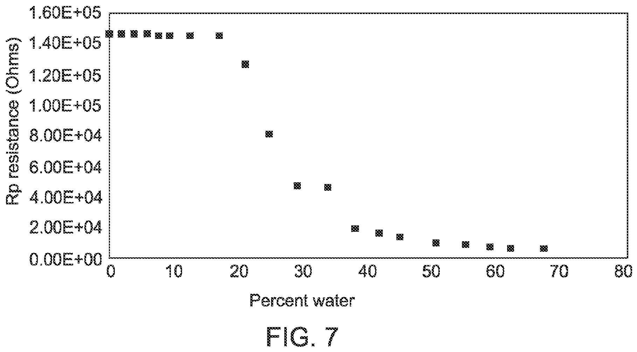

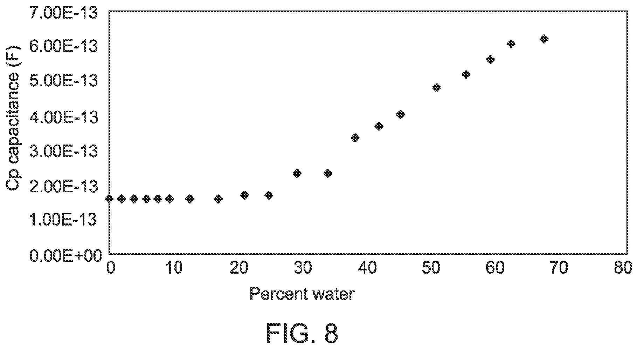

The three-dimensional resonant transducer 31 utilizes mutual inductance of the top winding 33 to sense the bottom winding 35. Illustrated in FIG. 6 is an equivalent circuit 41, including a current source 43, R0 resistor 45, C0 capacitor 47, and L0 inductor 49. The equivalent circuit 41 also includes L1 inductor 51, R1 resistor 53 and C1 capacitor 55. The circuit also includes Cp capacitor 57 and Rp resistor 59. The circled portion of the equivalent circuit 41 shows a sensitive portion 61 that is sensitive to the properties of the surrounding test fluid. A typical Rp response and Cp response of resonant a transducer 12 to varying mixtures of oil and water are shown in FIGS. 7 and 8 respectively.

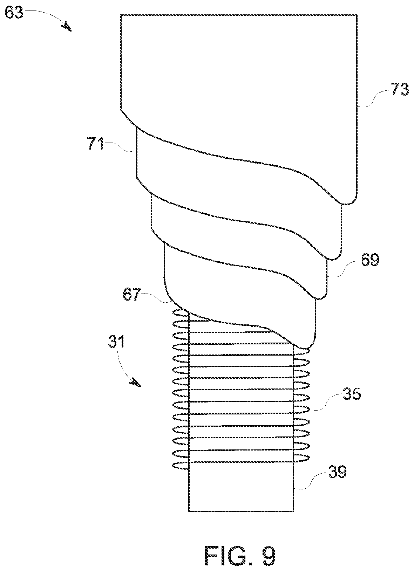

The three-dimensional resonant transducer 31 may be shielded as shown in FIG. 9. A resonant transducer assembly 63 includes a radio frequency absorber (RF absorber layer 67) surrounding the sampling cell 39, top winding 33, and bottom winding 35. A spacer 69 may be provided surrounded by a metal shield 71. The metal shield 71 is optional, and is not part of the transducer 31. The metal shield 71 allows operation inside or near metal objects and piping, reduces noise, and creates a stable environment such that any changes in the sensor response is directly due to changes in the test fluid. In order to successfully encapsulate the sensor in a metal shield 71 the RF absorber layer 67 may be placed between the sensor and the metal shield 71. This prevents the RF field from interacting with the metal and quenching the response of the sensor. The metal shield 71 may be wrapped with a cover 73 of suitable material. The RF absorber layer 67 can absorb electromagnetic radiation in different frequency ranges with non-limiting examples in the kilohertz, megahertz, gigahertz, terahertz frequency ranges depending on the operation frequency of the transducer 31 and the potential sources of interference. The absorber layer 67 can be a combination of individual layers for particular frequency ranges so the combinations of these individual layers provide a broader spectral range of shielding.

Fouling of the resonant sensor system 11 may be reduced by providing the resonant transducer 12 with a geometry that enables resonant transducer 12 to probe the environment over the sample depth perpendicular to the transducer ranging from 0.1 mm to 1000 mm. Signal processing of the complex impedance spectrum reduces the effects of fouling over the sample depth.

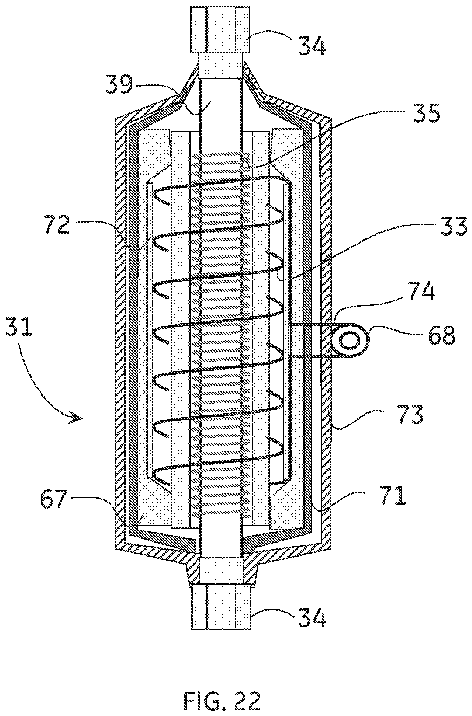

Shown in FIG. 22 is a second three-dimensional resonant transducer 31 according to the claimed invention. The second three-dimensional resonant transducer 31 includes a top winding 33 and a bottom winding 35. The bottom winding 35 is located around the sampling cell 39 and the top winding 33 is located around the bottom winding 35. The sampling cell 39 may, for example, be made of a material resistant to fouling and suitable for providing galvanic isolation between the bottom winding 35 and a fluid being sampled such as Polytetrafluoroethylene (PTFE), a synthetic fluoropolymer of tetrafluoroethylene. The sampling cell 39 may be in the form of a tube or otherwise adapted to contain a stationary or flowing fluid, typically a liquid. The fluid may comprise liquid or solid particles mixed with a liquid as in an emulsion, colloidal suspension, latex or other dispersion. A galvanic isolator is provided between the top winding 33 and the bottom winding 35. For example, the galvanic isolator may be a PTFE tube. The bottom winding 35 may be wound directly around a portion of the sampling cell 39 or otherwise fit around, or be in contact with, the outside of the sampling cell 39. The top winding 33 may be separated from the bottom winding 35 by a spacing of about 0.1" to 0.3". The top winding 33 and the bottom winding 35 are arranged as tubular coils concentric with each other and preferably with the sampling cell 39.

The second three-dimensional resonant transducer 31 has a spacer 72 between the top winding 33 and the RF absorber layer 67. The spacer 72 is made of galvanic isolating material. This spacer 72 increases signal while reducing noise resulting in a higher signal to noise ratio. The inventors have also observed that this spacer 72 can enhance the dynamic range of the second three-dimensional resonant transducer 31.

The second three-dimensional resonant transducer 31 has wires 74 connecting the ends of the top winding 33 to a connector 68. The connector 68 is used to connect an electrical cable from the analyzer 15 to the second three-dimensional resonant transducer 31. The second three-dimensional resonant transducer 31 also has fittings 34 at the ends of the sampling cell 39. The fittings 34 allow the sampling cell 39 to be optionally connected to one or more pipes, which may have valves or other flow control devices, adapted to bring a liquid sample into the sampling cell 39 and to remove a sample after it has been measured.

Optionally, the second three-dimensional resonant transducer 31 may have two galvanically isolated top windings 33, one that is used as a drive (excitation) coil and one that is used as a pick up (receiving) coil. However, in the example of FIG. 22, a single top winding 33 acts as both a drive coil and a pick up coil. Analyzer 15 is configured to both send current (a power wave) through the top winding 33 and to receive a signal (current) from the top winding but at different time intervals, for example according to an alternating pattern of excitation and receiving. The excitation and receiving steps may each have a duration of, for example, 0.2 to 5 seconds. The frequency of the power wave applied during the excitation stage may vary between successive excitation stages. In addition to avoiding a second top winding 33, this configuration avoids having two sets of electrical cables connecting the analyzer 15 to the second three-dimensional resonant transducer 31 and this tends to reduce signal noise.

The bottom winding 35 acts as a resonator or sensing coil. The bottom winding 35 floats with no galvanic connections to other parts of the second three-dimensional resonant transducer 31. The two ends of the bottom winding 35 are preferably not connected to each other (other than through the coils of the bottom winding 35) so as to form a circuit loop, although connections to form a circuit as in Figure 5, with or without a capacitor, may also be used. The bottom winding 35 is excited by an electro-magnetic field created by a power wave flowing through the top winding 33. The excited bottom winding 35 generates another electro-magnetic field that is altered by its interaction with the fluid in the sampling cell 39. This (reflected) electro-magnetic field is then and sensed by the top winding 33. As mentioned above, these two steps occur in different time periods, preferably repeated in alternation over a plurality of cycles.

Although the bottom winding 35 generates an electro-magnetic field, because the sampling cell 39 contains a fluid (such as water or oil) with low conductivity, signals representing the electric (as opposed to magnetic) portion of the field generated by the bottom winding 35 are the primary or only means of analysis. This is in contrast to eddy current techniques used when making measurements of more conductive materials that use the magnetic portion of a field generated by a resonator as the primary or only means of analysis. Signals associated with the magnetic portion of the electro-magnetic field generated by the bottom winding 35 would tend to indicate the conductivity of a sample whereas signals associated with the electric portion of the electro-magnetic field generated by the bottom winding 35 indicate the impedance of the sample.

The analyzer 15 translates the electric response (signal) generated by the bottom winding 33 (as received through the top winding 35) into one or more measured parameters. These parameters may include one or more of: complex (magnitude and phase) impedance response; resonance peak position, peak width, peak height and/or peak symmetry of the impedance response; magnitude of the real part of the impedance; resonant frequency of the imaginary part of the impedance; antiresonant frequency of the imaginary part of the impedance; zero-reactance frequency; phase angle of impedance; magnitude of impedance; and, others.

The second three-dimensional resonant transducer 31 of FIG. 22 may be used in any method or apparatus described for the resonant transducer 31 of FIG. 5. The second three-dimensional resonant transducer 31 of FIG. 22 utilizes mutual inductance of the top winding 33 to sense the bottom winding 35. The equivalent circuit in FIG. 6 may be used with the second three-dimensional resonant transducer 31 of FIG. 22. An Rp response and Cp response to varying mixtures of oil and water similar to that shown in FIGS. 7 and 8 respectively may be obtained from the second three-dimensional resonant transducer 31 of FIG. 22.

The second three-dimensional resonant transducer 31 may be shielded as shown in FIG. 22. A resonant transducer assembly 63 includes a radio frequency absorber (RF absorber layer 67) surrounding the sampling cell 39, top winding 33, and bottom winding 35. The RF absorber layer 67 may be surrounded by a metal, for example aluminum, shield 71. There may be a spacer (not shown) between the RF absorber layer 67 and the shield 71. The shield 71 is optional, and is not a necessary part of the second three-dimensional resonant transducer 31. However, the shield 71 improves operation inside or near metal objects and piping, reduces noise, and creates a stable environment such that any changes in the sensor response is directly due to changes in the test fluid. In order to successfully encapsulate the sensor in a shield 71 the RF absorber layer 67 may be placed between the sensor and the metal shield 71. This prevents the RF field from interacting with the metal and quenching the response of the sensor. The metal shield 71 may be wrapped with a cover 73 of suitable material. The RF absorber layer 67 can absorb electromagnetic radiation in different frequency ranges with non-limiting examples in the kilohertz, megahertz, gigahertz, terahertz frequency ranges depending on the operation frequency of the transducer 31 and the potential sources of interference. The absorber layer 67 can be a combination of individual layers for particular frequency ranges so the combinations of these individual layers provide a broader spectral range of shielding.

The top winding 33 is preferably, but not necessarily, at least half as long as the bottom winding 35. The top winding 33 preferably, but not necessarily, has a larger pitch than the bottom winding 35. For example, as shown in in FIG. 22, the top winding 33 is about as long as the bottom winding 35 but has less than one tenth as many turns as the bottom winding 35. For example, the top winding 33 may have one turn for every 15 to 50 turns of the bottom winding 35. The top winding 33 and the bottom winding 35 have different resonant frequencies. When measuring the concentration of water in oil or oil in water, or the concentration of salts or solid particles in a water or oil or water and oil based mixtures, the top winding 33 preferably has a higher resonant frequency than the bottom winding 35. The resonant frequencies of the top winding 33 and the bottom winding 35 are preferably baseline separated. Successive peaks of the applied and reflected (modified by interaction with the sample) signals are separated by at least some distance along the baseline.

The concentric arrangement of the top winding 33 and the bottom winding 35 shown in FIG. 22 increases the sensitivity of the second three-dimensional resonant transducer 31. For example, the second three-dimensional resonant transducer 31 of FIG. 22 may be better able to determine the composition of emulsions and other dispersions, including dispersions of solid particles and dispersion containing both solid particles and an emulsion, compared to the resonant transducer 31 of FIG. 5.

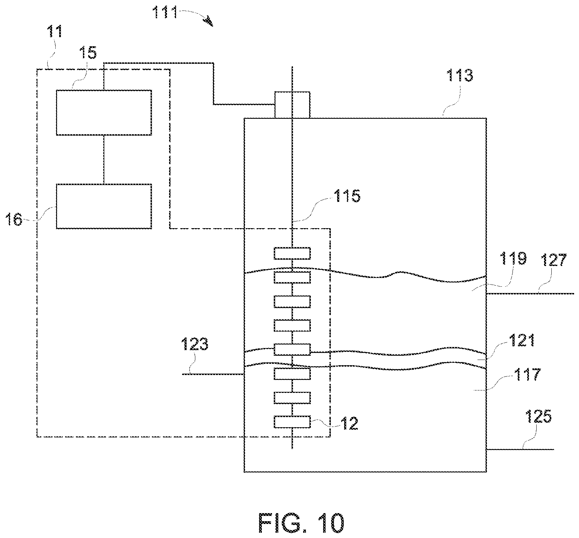

As shown in FIG. 10, the resonant sensor system 11 may be used to determine the level and composition of fluids in a fluid processing system 111. Fluid processing system 111 includes a vessel 113 with a sampling assembly 115 and a resonant sensor system 11. The resonant sensor system 11 includes at least one resonant transducer 12 coupled to the sampling assembly 115. Resonant sensor system 11 also includes an analyzer 15 and a processor 16.

In operation, a normally immiscible combination of fluids enters the vessel through a raw fluid input 123. The combination of fluids may include a first fluid and a second fluid normally immiscible with the first fluid. As the combination of fluids is processed, the combination of fluids is separated into a first fluid layer 117, and a second fluid layer 119. In between the first fluid layer 117 and second fluid layer 119, there may be a rag layer 121. After processing, a first fluid may be extracted through first fluid output 125, and a second fluid may be extracted through second fluid output 127. The resonant sensor system 11 is used to measure the level of the first fluid layer 117, the second fluid layer 119and the rag layer 121. The resonant sensor system 11 may also be used to characterize the content of the first fluid layer 117, the second fluid layer 119 and the rag layer 121.

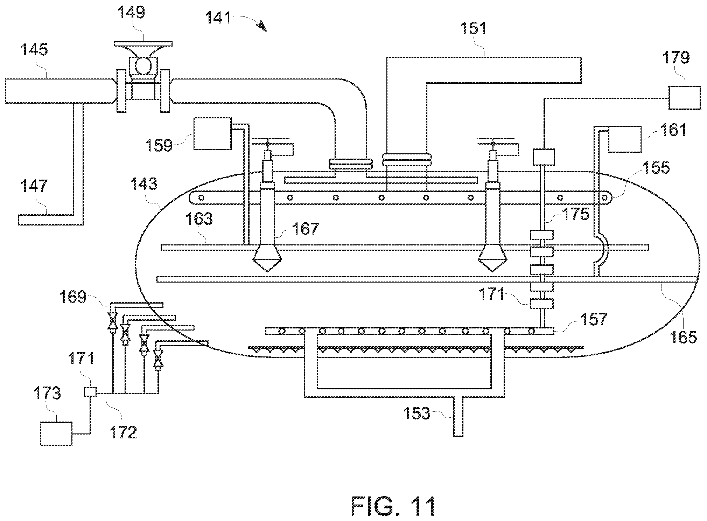

An embodiment of a fluid processing system 111 is a desalter 141 illustrated in FIG. 11. The desalter 141 includes a desalter vessel 143. Raw oil enters the desalter 141 through crude oil input 145 and is mixed with water from water input 147. The combination of crude oil and water flows through mixing valve 149 and into the desalter vessel 143. The desalter 141 includes a treated oil output 151 and a wastewater output 153. Disposed within the desalter vessel 143 are an oil collection header 155 and a water collection header 157. Transformer 159 and transformer 161 provide electricity to top electrical grid 163 and bottom electrical grid 165. Disposed between top electrical grid 163 and bottom electrical grid 165 are emulsion distributors 167.

In operation, crude oil mixed with water enters the desalter vessel 143 and the two fluids are mixed and distributed by emulsion distributors 167 thereby forming an emulsion. The emulsion is maintained between the top electrical grid 163 and the bottom electrical grid 165. Salt containing water is separated from the oil/water mixture by the passage through the top electrical grid 163 and bottom electrical grid 165 and drops towards the bottom of the desalter vessel 143 where it is collected as waste water.

Control of the level of the emulsion layer and characterization of the contents of the oil-in-water and water-in-oil emulsions is important in the operation of the desalter 141. Determination of the level of the emulsion layer may be accomplished using a sampling assembly such as a try-line assembly 169 coupled to the desalter vessel 143 and having at least one resonant transducer 12 disposed on try-line output conduit 172. The resonant transducer 12 may be coupled to a data collection component 173. In operation, the resonant transducer 12 is used to measure the level of water and the oil and to enable operators to control the process. The try-line assembly 169 may be a plurality of pipes open at one end inside the desalter vessel 143 with an open end permanently positioned at the desired vertical position or level in the desalter vessel 143 for withdrawing liquid samples at that level. There are generally a plurality of sample pipes in a processing vessel, each with its own sample valve, with the open end of each pipe at a different vertical position inside the unit, so that liquid samples can be withdrawn from a plurality of fixed vertical positions in the unit. Another approach to measuring the level of the emulsion layer is to use a swing arm sampler. A swing arm sampler is a pipe with an open end inside the desalter vessel 143 typically connected to a sampling valve outside the unit. It includes an assembly used to change the vertical position of the open end of the angled pipe in the desalter 141, by rotating it, so that liquid samples can be withdrawn (or sampled) from any desired vertical position.

Another method to measure the level of the oil and water is to dispose at least one resonant transducer 12 on a dipstick 175. A dipstick 175 may be a rod with a resonant transducer 12 that is inserted into the desalter vessel 143. Measurements are made at a number of levels. Alternately, the dipstick 175 may be a stationary rod having a plurality of multiplexed resonant transducers 12. The resonant transducer 12 may be coupled to a data collection component 179 that collects data from the various readings for further processing.

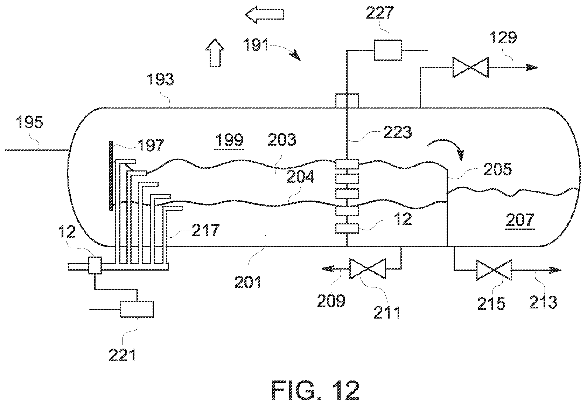

Another embodiment of a fluid processing system 111 is a separator 191 illustrated in FIG. 12. The separator 191 includes a separator vessel 193 having an input conduit 195 for crude oil. Crude oil flowing from input conduit 195 impacts an inlet diverter 197. The impact of the crude oil on the inlet diverter 197 causes water particles to begin to separate from the crude oil. The crude oil flows into the processing chamber 199 where it is separated into a water layer 201 and an oil layer 203. The crude oil is conveyed into the processing chamber 199 below the oil/water interface 204. This forces the inlet mixture of oil and water to mix with the water continuous phase in the bottom of the vessel and rise through the oil/water interface 204 thereby promoting the precipitation of water droplets which are entrained in the oil. Water settles to the bottom while the oil rises to the top. The oil is skimmed over a weir 205 where it is collected in oil chamber 207. Water may be withdrawn from the system through a water output conduit 209 that is controlled by a water level control valve 211. Similarly oil may be withdrawn from the system through an oil output conduit 213 controlled by an oil level control valve 215. The height of the oil/water interface may be detected using a try-line assembly 217 having at least one resonant transducer 12 disposed in a try-line output conduit 218 and coupled to a data processor 221. Alternately a dip stick 223 having at least one resonant transducer 12 coupled to a processor 227 may be used to determine the level of the oil/water interface 204. The determined level is used to control the water level control valve 211 to allow water to be withdrawn so that the oil/water interface is maintained at the desired height.

The following examples are given by way of illustration only and are not intended as a limitation of the scope of this disclosure. A model system of heavy mineral oil, tap water and detergent was used to carry out static tests for various designs of resonant transducer 12. The level of detergent was kept constant for all of the mixtures.

### EXAMPLE 1

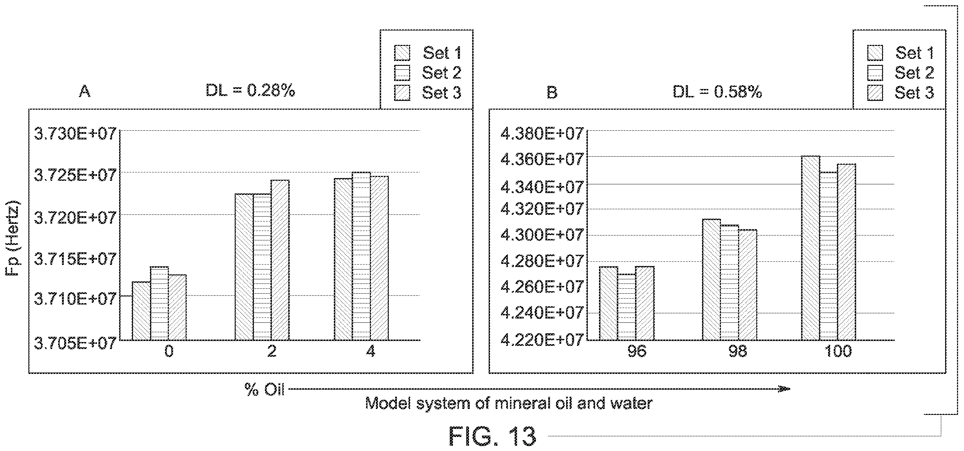

In the case of the three-dimensional resonant transducer 31 disposed on a try-line or swing arm sampling assembly 13, different compositions of oil and water were poured into a sample cell with the three-dimensional resonant transducer 31 wound around the outside of the sample cell. FIG. 13 shows the try-line/swing arm response in terms of Fp (frequency shift of the real impedance) as oil concentration increases. The calculated detection limit of the composition of oil in oil-in-water emulsions (FIG. 13 part A) is 0.28% and of oil in water-in-oil emulsions (FIG. 13 part B) is 0.58%.

### EXAMPLE 2

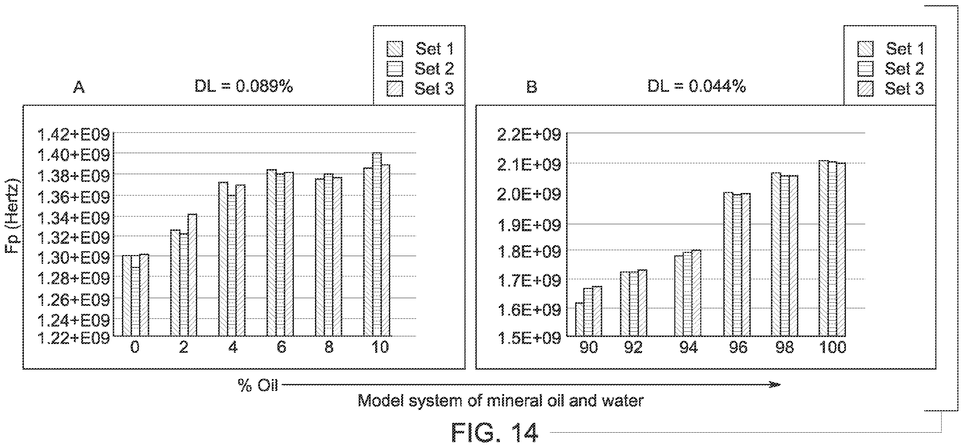

In the case of the two-dimensional resonant transducer 25, the two-dimensional resonant transducer 25 was immersed in different compositions of oil and water. FIG. 14 shows the response of a two-dimensional resonant transducer 25 (2 cm circular) in terms of Fp (frequency shift of the real impedance) as oil concentration increases. The calculated detection limit of the composition of oil in oil-in-water emulsions (FIG. 14 part A) is 0.089% and of oil in water-in-oil emulsions (FIG. 14 part B) is 0.044%. This example illustrates that small concentrations of one fluid mixed large concentrations of another fluid can be measured with a high degree of accuracy.

### EXAMPLE 3

The model system was loaded with 250 mL of mineral oil and treated with detergent at a concentration of 1 drop per 50 mL (5 drops). The mineral oil was stirred and injected through the sensor and the impedance spectra are recorded. Small additions of water were added with constant salinity and same detergent treatment. After the water volume exceeded 66% or 500 mL of water, the system was cleaned and the experiment is repeated with different salinity waters. The multivariate response of the two-dimensional resonant transducer 25 was sensitive to changes in composition and conductivity at all levels in the test vessel of the model system. Although the effect of conductivity and composition are somewhat convoluted, the fact that the sensor monitors a composition gradient allows the data analysis procedure to deconvolute these effects.

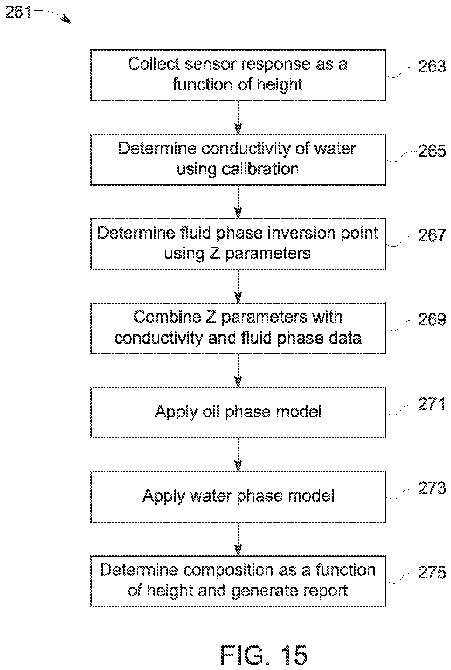

FIG. 15 is a generalized process diagram illustrating a method 261 for determining the composition of an oil and water mixture as a function of height.

In step 263 data (a set of LCR resonant circuit parameters) is collected as a function of height from top to bottom (in the lab, this is simulated by starting with 100% oil and gradually adding water).

In step 265 the conductivity of water using calibration is determined At 100% water, the multivariate response is compared to a calibration for water conductivity.

In step 267 the fluid phase inversion point is determined using Z parameters.

In step 269 the Z parameters are combined with conductivity and fluid phase data.

In step 271 an oil phase model is applied. The oil phase model is a set of values correlating measured frequency values, impedance values and conductivity values to oil content in an oil and water mixture.

In step 273 a water phase model is applied. The water phase model is a set of values correlating measured frequency values, impedance values and conductivity values to water content in a water and oil mixture.

In step 275 the composition as a function of height is determined using the conductivity and the fluid phase inversion point as input parameters in the multivariate analysis and a report is generated.

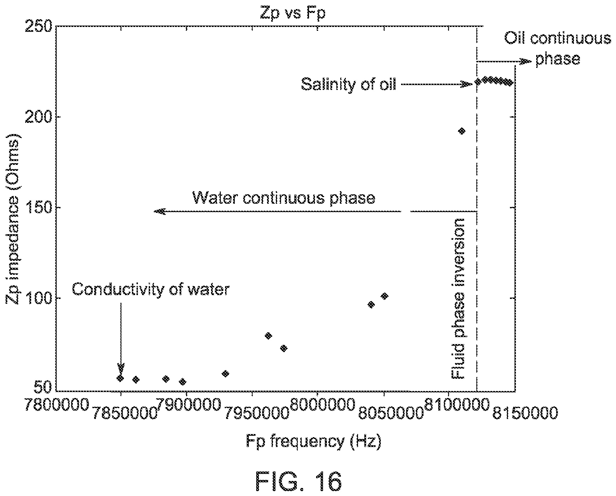

FIG. 16 shows the raw impedance (Zp) vs. frequency (Fp) data for a profile containing 0-66% water from right to left. At approximately 8.12 MHz, the water content is high enough (-25%) to induce fluid phase inversion from oil to water continuous phase. This is apparent from the drastic change in Zp due to the increased conductivity of the test fluid in water continuous phase. An oil continuous phase model is applied to any data points to the right of the fluid phase inversion and a water model to the left. Additionally, a calibration is applied to the endpoint to determine the conductivity of the water, which in this case was 2.78 mS/cm.

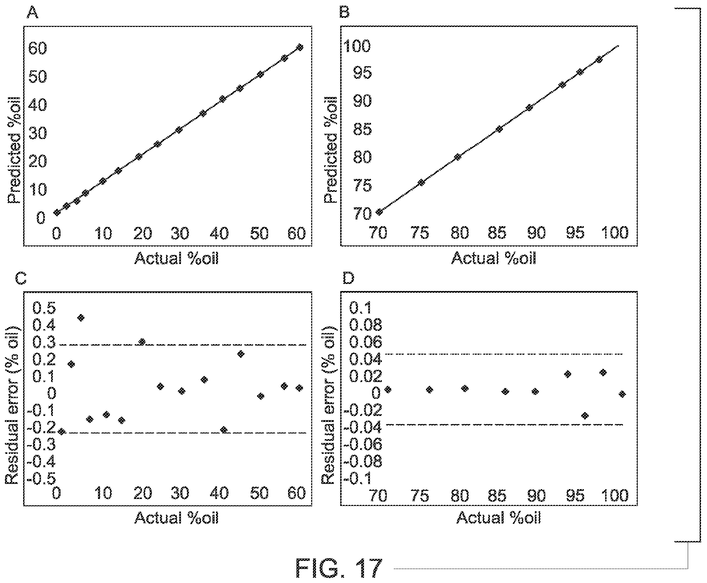

FIG. 17 shows the results of an analysis of the experiment data from an embodiment of a three-dimensional resonant sensor system illustrated the correlation between the actual and predicted values of oil in water and water in oil and the residual errors of prediction based on developed model. Part A of the chart plots the actual and predicted values of oil in water. Part B of the chart plots the actual and predicted values of water in oil. In part A, the data points were modeled separately from the data points in part B (water continuous phase). Parts C and D of the chart plot the residual error between the actual and predicted values of oil in water and water in oil respectively. Generally, the residual error was less than 0.5% when the actual percentage of oil is between 0% to 60%. The residual error was less than 0.04% when the actual percentage of oil is between 70% to 100%. At the fluid phase inversion the residual error increases up to 10% where prediction capability is difficult due to fluctuations in the composition of the test fluid in the dynamic test rig. The prediction capability of the sensor will improve at compositions >66% water with more training data.

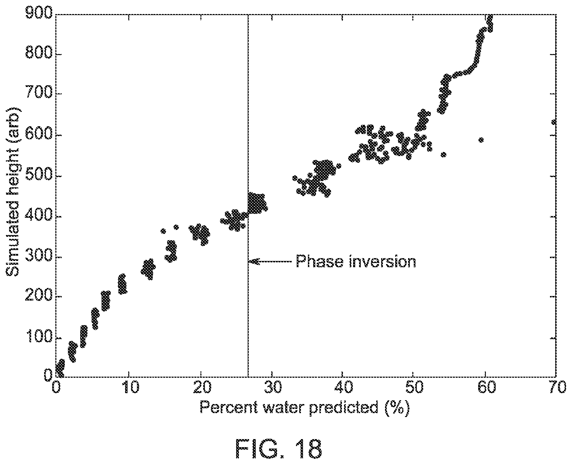

FIG. 18 illustrates the results obtained in a simulated desalter. The chart shows a profile developed by plotting the composition as a function of time. To simulate the sampling using a swing arm that is slowly rotated through the rag layer, a test rig was operated such that the composition of the test fluid was slowly modulated with time by adding small additions of water.

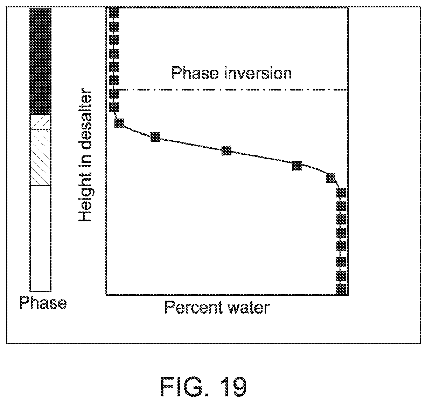

FIG. 19 is an illustration of the expected level of reporting from the sensor data analysis system. The end user will be shown a plot that displays a representation of the composition as a function of height in the desalter, the level of fluid phase inversion, and the width of the rag layer. On the left are fluid phase indicators (black-oil, gray-oil continuous, cross hatched-water continuous, white-water) that indicate the percent water/height curve. The height of the rag layer is the sum of the water continuous and oil continuous regions. The level of detail indicated will allow the operator of the desalter to optimize the feed rate of chemicals into the process, provide more detailed feedback on the performance of a fluid processing system, and highlight process upsets that may cause damage to downstream process infrastructure.

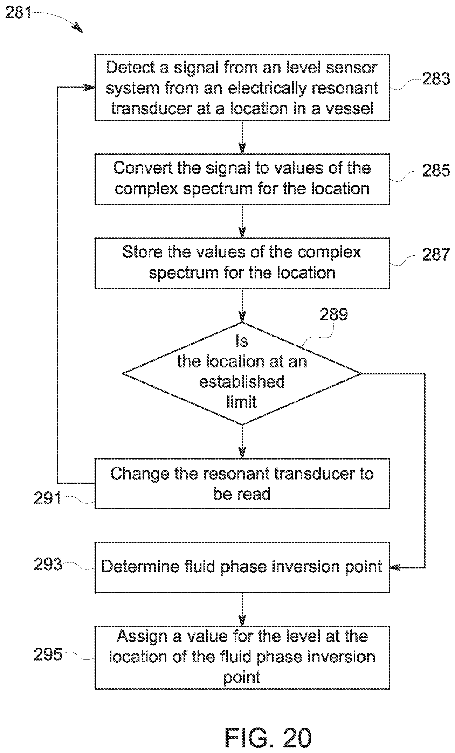

Illustrated in FIG. 20 is a method 281 for measuring the level of a mixture of fluids in a vessel 113.

In step 283, the method 281 may detect signals (a set of signals) from a resonant sensor system 11 at a plurality of locations in a vessel. The signals are generated by a resonant transducer 12 immersed in the mixture of fluids. The resonant transducer 12 generates a set of transducer signals corresponding to changes in dielectric properties of the resonant transducer 12, and the signals are detected by an analyzer 15.

In step 285, the method 281 may convert the signals to a set of values of the complex impedance spectrum for the plurality of locations. The conversion is accomplished using multivariate data analysis.

In step 287, the method 281 may store the values of the complex impedance spectrum.

In step 289, the method 281 may determine if a sufficient number of locations have been measured.

In step 291, the method 281 may change the resonant transducer 12 being read (or the location of the resonant transducer 12) if an insufficient number of locations have been measured.

In step 293, the method 281 may determine the fluid phase inversion point if a sufficient number of locations has been measured. The fluid phase inversion point is determined from the values of the complex impedance spectrum by identifying a drastic change in the impedance values.

In step 295, the method 281 may assign a value for the interface level based on the fluid phase inversion point.

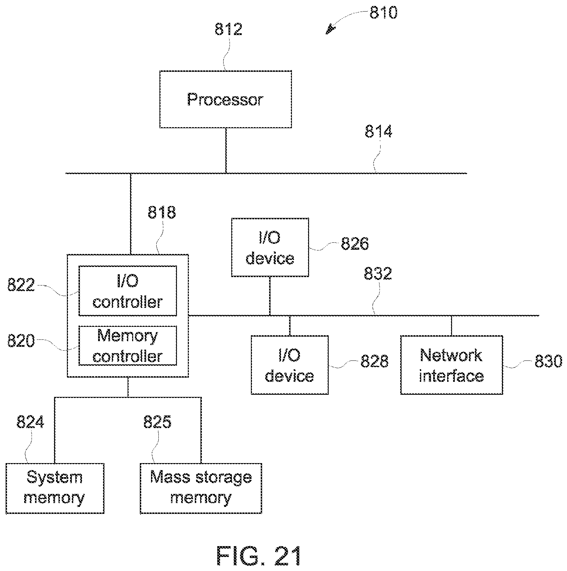

FIG. 21 is a block diagram of non-limiting example of a processor system 810 that may be used to implement the apparatus and methods described herein. As shown in FIG. 21, the processor system 810 includes a processor 812 that is coupled to an interconnection bus 814. The processor 812 may be any suitable processor, processing unit or microprocessor. Although not shown in FIG. 21, the processor system 810 may be a multi-processor system and, thus, may include one or more additional processors that are identical or similar to the processor 812 and that are communicatively coupled to the interconnection bus 814.

The processor 812 of FIG. 21 is coupled to a chipset 818, which includes a memory controller 820 and an input/output (I/O) controller 822. As is well known, a chipset typically provides I/O and memory management functions as well as a plurality of general purpose and/or special purpose registers, timers, etc. that are accessible or used by one or more processors coupled to the chipset 818. The memory controller 820 performs functions that enable the processor 812 (or processors if there are multiple processors) to access a system memory 824 and a mass storage memory 825.

The system memory 824 may include any desired type of volatile and/or nonvolatile memory such as, for example, static random access memory (SRAM), dynamic random access memory (DRAM), flash memory, read-only memory (ROM), etc. The mass storage memory 825may include any desired type of mass storage device including hard disk drives, optical drives, tape storage devices, etc.

The I/O controller 822 performs functions that enable the processor 812 to communicate with peripheral input/output (I/O) devices 826 and 828 and a network interface 830 via an I/O bus 832. The I/O devices 826 and 828 may be any desired type of I/O device such as, for example, a keyboard, a video display or monitor, a mouse, etc. The I/O devices 826 and 828 also may be The network interface 830 may be, for example, an Ethernet device, an asynchronous transfer mode (ATM) device, an 802.11 device, a DSL modem, a cable modem, a cellular modem, etc. that enables the processor system 810 to communicate with another processor system. Data from analyzer 15 may be communicated to the processor 812 through the I/O bus 832 using the appropriate bus connectors.

While the memory controller 820 and the I/O controller 822 are depicted in FIG. 21 as separate blocks within the chipset 818, the functions performed by these blocks may be integrated within a single semiconductor circuit or may be implemented using two or more separate integrated circuits.

Certain embodiments contemplate methods, systems and computer program products on any machine-readable media to implement functionality described above. Certain embodiments may be implemented using an existing computer processor, or by a special purpose computer processor incorporated for this or another purpose or by a hardwired and/or firmware system, for example. Certain embodiments include computer-readable media for carrying or having computer-executable instructions or data structures stored thereon. Such computer-readable media may be any available media that may be accessed by a general purpose or special purpose computer or other machine with a processor. By way of example, such computer-readable media may comprise RAM, ROM, PROM, EPROM, EEPROM, Flash, CD-ROM or other optical disk storage, magnetic disk storage or other magnetic storage devices, or any other medium which can be used to carry or store desired program code in the form of computer-executable instructions or data structures and which can be accessed by a general purpose or special purpose computer or other machine with a processor. Combinations of the above are also included within the scope of computer-readable media. Computer-executable instructions comprise, for example, instructions and data which cause a general purpose computer, special purpose computer, or special purpose processing machines to perform a certain function or group of functions.

Generally, computer-executable instructions include routines, programs, objects, components, data structures, etc., that perform particular tasks or implement particular abstract data types. Computer-executable instructions, associated data structures, and program modules represent examples of program code for executing steps of certain methods and systems disclosed herein. The particular sequence of such executable instructions or associated data structures represent examples of corresponding acts for implementing the functions described in such steps.

Embodiments of the present disclosure may be practiced in a networked environment using logical connections to one or more remote computers having processors. Logical connections may include a local area network (LAN) and a wide area network (WAN) that are presented here by way of example and not limitation. Such networking environments are commonplace in office-wide or enterprise-wide computer networks, intranets and the Internet, and may use a wide variety of different communication protocols. Those skilled in the art will appreciate that such network-computing environments will typically encompass many types of computer system configurations, including personal computers, handheld devices, multi-processor systems, microprocessor-based or programmable consumer electronics, network PCs, minicomputers, mainframe computers, and the like. Embodiments of the disclosure may also be practiced in distributed computing environments where tasks are performed by local and remote processing devices that are linked (either by hardwired links, wireless links, or by a combination of hardwired or wireless links) through a communications network. In a distributed computing environment, program modules may be located in both local and remote memory storage devices.

Monitoring changes of the complex impedance of the circuit and applying chemometric analysis of the impedance spectra allows for the composition and continuous phase of oil-in-water and water-in-oil mixtures to be predicted with a standard error of 0.04% in 0-30% water and 0.26% in 30-100% water.

Multivariate analysis tools in combination with data-rich impedance spectra allow for elimination of interferences, and transducers designed for maximum penetration depth decreases the impact of fouling. As the penetration depth of the resonator is extended further into the bulk of the fluid, surface fouling becomes less significant.

The term "analyte" includes any desired measured environmental parameter.

The term "environmental parameters" is used to refer to measurable environmental variables within or surrounding a manufacturing or monitoring system. The measurable environmental variables comprise at least one of physical, chemical and biological properties and include, but are not limited to, measurement of temperature, pressure, material concentration, conductivity, dielectric property, number of dielectric, metallic, chemical, or biological particles in the proximity or in contact with the sensor, dose of ionizing radiation, and light intensity.

The term "fluids" includes gases, vapors, liquids, and solids.

The term "interference" includes any undesired environmental parameter that undesirably affects the accuracy and precision of measurements with the sensor. The term "interferent" refers to a fluid or an environmental parameter (that includes, but is not limited to temperature, pressure, light, etc.) that potentially may produce an interference response by the sensor.

The term "transducer" means a device that converts one form of energy to another.

The term "sensor" means a device that measures a physical quantity and converts it into a signal which can be read by an observer or by an instrument.

The term "multivariate data analysis" means a mathematical procedure that is used to analyze more than one variable from a sensor response and to provide the information about the type of at least one environmental parameter from the measured sensor spectral parameters and/or to quantitative information about the level of at least one environmental parameter from the measured sensor spectral parameters.

The term "resonance impedance" or "impedance" refers to measured sensor frequency response around the resonance of the sensor from which the sensor "spectral parameters" are extracted.

The term "spectral parameters" is used to refer to measurable variables of the sensor response. The sensor response is the impedance spectrum of the resonance sensor circuit of the resonant transducer 12. In addition to measuring the impedance spectrum in the form of Z-parameters, S-parameters, and other parameters, the impedance spectrum (both real and imaginary parts) may be analyzed simultaneously using various parameters for analysis, such as, the frequency of the maximum of the real part of the impedance (Fp), the magnitude of the real part of the impedance (Zp), the resonant frequency of the imaginary part of the impedance (F 1), and the anti-resonant frequency of the imaginary part of the impedance (F 2), signal magnitude (Z 1) at the resonant frequency of the imaginary part of the impedance (F 1), signal magnitude (Z 2) at the anti-resonant frequency of the imaginary part of the impedance (F 2), and zero-reactance frequency (Fz), frequency at which the imaginary portion of impedance is zero). Other spectral parameters may be simultaneously measured using the entire impedance spectra, for example, quality factor of resonance, phase angle, and magnitude of impedance. Collectively, "spectral parameters" calculated from the impedance spectra, are called here "features" or "descriptors". The appropriate selection of features is performed from all potential features that can be calculated from spectra. Multivariable spectral parameters are described in U.S. patent application, Ser. No. 12/118,950 entitled "Methods and systems for calibration of RFID sensors".

The terminology used herein is for the purpose of describing particular embodiments only and is not intended to be limiting of the invention. Where the definition of terms departs from the commonly used meaning of the term, applicant intends to utilize the definitions provided herein, unless specifically indicated. The singular forms "a", "an" and "the" are intended to include the plural forms as well, unless the context clearly indicates otherwise. It will be understood that, although the terms first, second, etc. may be used to describe various elements, these elements should not be limited by these terms. These terms are only used to distinguish one element from another. The term "and/or" includes any, and all, combinations of one or more of the associated listed items. The phrases "coupled to" and "coupled with" contemplates direct or indirect coupling.

In some embodiments, the present invention uses the electric field and a single resonant coil that is capable of quantifying a large dynamic range, for example of 0-100% water, and characterizing the continuous phase of oil/water emulsions observed. Multiple sensing coils are not required to cover the broad dynamic range exhibited by fluids that are either oil/gas or water continuous phase. Without intending to be limited by theory, the ability to operate with a single sensing coil results from not using an eddy current based method wherein the power loss or attenuation of a magnetic field is determined and correlated to the conductive component content of a multiphase fluid.

Similarly, in at least some embodiments, the present invention does not require a combination of an eddy current or other transducer with a low frequency capacitance probe (or separate sensors to probe capacitance and conductance generally) in order to differentiate the complexity of the samples. According to the present invention, only a single sensing coil and a second coil that both transmits and receives the signal are required.

In at least some embodiments of the present invention, sensing measurements are performed over a broad range of frequencies, where the range of frequencies includes regions where the resonator signal may be only 10%, 1% or even 0.001% from its maximum response. Sensing methods may include one or more of (1) to scan the sensor response over the where the range of frequencies includes regions where the resonator signal is only 0.001 - 10% from its maximum response, (2) to analyze the collected spectrum for the simultaneous changes to one or more of a number of measured parameters that included the resonance peak position, magnitude of the real part of the impedance, resonant frequency of the imaginary part of the impedance, antiresonant frequency of the imaginary part of the impedance, and others, (3) to determine the composition of fluid mixtures even when one of the fluids is at a low concentration, and (4) to determine fluid level and to determine emulsion layer. Spectrum information that is both slightly lower and higher in resonant frequency may be used. Optionally, a single coil may accomplish two functions - excitation and receiving signal, optionally simultaneously.

At least some embodiments of the present invention employ two coils with resonant frequencies with baseline separation between the frequency bands. In this way, the intrinsic resonant signal of the pick-up coil (which may be used as both the transmission and receiving coil) does not influence the resonance signal of the sensing coil.

## Claims

1. A sensor comprising:
a three-dimensional resonant transducer (31) configured to determine a composition of an emulsion or other dispersion,
wherein the three-dimensional resonant transducer comprises:
- a sampling cell (39);
- a top winding (33) and a bottom winding (35), the bottom winding (35) being located around the sampling cell (39) and the top winding (33) being located around and concentric to the bottom winding (35);
- a galvanic isolator provided between the top winding (33) and the bottom winding (35) ;
- a radio-frequency (RF) absorber layer (67) disposed around the top winding (33);
- a spacer (72) disposed between the top winding (33) and the RF absorber layer (67); the spacer (72) being made of galvanic isolating material;
- wires (74) connecting the ends of the top winding (33) to a connecter (68), said connecter being adapted to connect an electrical cable from an impedance analyzer (15) to the three-dimensional resonant transducer (31);
- fittings (34) at the ends of the sampling cell (39);
wherein:
the top winding (33) is adapted to act as both a drive coil and a pick up coil;
the bottom winding (35) is adapted to act as a resonator or sensing coil,
the bottom winding (35) is a floating winding, with no galvanic connections to other parts of the three-dimensional resonant transducer (31);
the bottom winding (35) is adapted to be excited by an electro-magnetic field created by a power wave flowing through the top winding (33) such that the excited bottom winding (35) generates another electro-magnetic field that is altered by its interaction with the fluid in the sampling cell (39), said altered electro-magnetic field being then sensed by the top winding (33).

2. The sensor of claim 1 wherein the RF absorber (67) absorbs electromagnetic radiation in the kilohertz, megahertz, gigahertz, and terahertz frequency ranges depending on the operation frequency of the three-dimensional resonant transducer (31) and the potential source of interferences.

3. The sensor of claim 1 wherein the RF absorber (67) is a combination of individual layers for particular frequency ranges.

4. The sensor of any one of claims 1 to 3 wherein the top winding (33) is at least half as long as the bottom winding (35).

5. The sensor of any claims 1 to 4 wherein the top winding (33) has a greater pitch than the bottom winding (35).

6. The sensor of any claims 1 to 5, the three-dimensional resonant transducer (31) further comprising a metal shield (71) around the RF absorber (67).

7. A sensor system (11) comprising
a sensor according to any one of claims 1 to 6;
a sampling assembly (13); and
an impedance analyzer (15); wherein the impedance analyzer is connected only to the top winding (33) of the sensor.

8. The sensor system of claim 7 wherein:
the resonant transducer (31) comprises a resonator configured to measure a set of LCR resonant circuit parameters; and
the set of LCR resonant circuit parameters comprise at least one of an impedance spectrum, a real part of the impedance spectrum, an imaginary part of an impedance spectrum, both real and imaginary parts of the impedance spectrum, a frequency of a maximum of the real part of a complex impedance, a magnitude of the real part of the complex impedance, a resonant frequency, a magnitude of the imaginary part of the complex impedance, and an anti-resonant frequency and a magnitude of the imaginary part of the complex impedance.

9. The sensor system of claim 8 wherein the impedance analyzer (15) converts the set of LCR resonant circuit parameters to values of a complex impedance spectrum.

10. A sensor system for determining a composition of a mixture of oil and water in a vessel comprising:
a subsystem that determines a set of complex impedance spectrum values of the of oil at one end of the vessel and the water at the opposite end with a sensor according to any one of claims 1 to 6;
a subsystem that generates calibration values for the sensor system for 100% oil and 100% water, respectively;
a subsystem that generates a model from the calibration values; and
a subsystem that applies the model to the set of complex impedance spectrum values to determine the composition.

11. A method for measuring an interface height between fluids in a vessel, the method comprising,
detecting a set of signals from a sensor according to any of claims 1 to 6 at a plurality of locations in the vessel;
converting the set of signals to values related to impedance of sampled fluid for the plurality of locations; and,
determining a fluid phase inversion point from the values.

12. A method for determining a composition of a mixture of particles in a liquid comprising
determining with a sensor system (11) according to any of claims 7-10 a value related to the impedance of the mixture; and,
applying a phase model of the liquid to the value.

## Patentansprüche

1. Sensor, umfassend:
einen dreidimensionalen Resonanzwandler (31), der konfiguriert ist, um eine Zusammensetzung einer Emulsion oder anderen Dispersion zu bestimmen,
wobei der dreidimensionale Resonanzwandler umfasst:
- eine Probenentnahmezelle (39);
- eine obere Wicklung (33) und eine untere Wicklung (35), wobei die untere Wicklung (35) um die Probenentnahmezelle (39) herum angeordnet ist und die obere Wicklung (33) um die untere Wicklung (35) herum und konzentrisch dazu angeordnet ist;
- einen galvanischen Isolator, der zwischen der oberen Wicklung (33) und der unteren Wicklung (35) vorgesehen ist;
- eine Funkfrequenz (HF) Absorber-Schicht (67), die um die obere Wicklung (33) herum angeordnet ist;
- ein Abstandsstück (72), das zwischen der oberen Wicklung (33) und der HF Absorber-Schicht (67) angeordnet ist; wobei das Abstandsstück (72) aus einem galvanischen Isolationsmaterial gebildet ist;
- Drähte (74), die die Enden der oberen Wicklung (33) mit einem Verbinder (68) verbinden, wobei der Verbinder ausgelegt ist, um ein elektrisches Kabel von einem Impedanz-Analysator (15) mit dem dreidimensionalen Resonanzwandler (31) zu verbinden;
- Beschläge (34) an den Enden der Probenentnahmezelle (39);
wobei:
die obere Wicklung (33) ausgelegt ist, um sowohl als eine Ansteuerspule als auch eine Aufnahmespule zu arbeiten;
die untere Wicklung (35) ausgelegt ist, um als eine Resonator- oder Erfassungsspule zu arbeiten,
die untere Wicklung (35) eine schwebende Wicklung ist, mit keinen galvanischen Verbindungen zu anderen Teilen des dreidimensionalen Resonanzwandlers (31);
die untere Wicklung (35) ausgelegt ist, um durch ein elektromagnetisches Feld angeregt zu werden, welches durch eine Energiewelle, die durch die obere Wicklung (33) fließt, erzeugt wird, so dass die angeregte untere Wicklung (35) ein anderes elektromagnetisches Feld erzeugt, das durch seine Wechselwirkung mit dem Fluid in der Probenentnahmezelle (39) verändert wird, wobei das veränderte elektromagnetische Feld dann von der oberen Wicklung (33) erfasst wird.

2. Sensor nach Anspruch 1, wobei der HF Absorber (67) eine elektromagnetische Strahlung in den Kilohertz, Megahertz, Gigahertz und Terahertz-Frequenzbereichen in Abhängigkeit von der Betriebsfrequenz des dreidimensionalen Resonanzwandlers (31) und der potentiellen Störungsquellen absorbiert.

3. Sensor nach Anspruch 1, wobei der HF Absorber (67) eine Kombination von einzelnen Schichten für bestimmte Frequenzbereiche ist.

4. Sensor nach einem der Ansprüche 1 bis 3, wobei die obere Wicklung (33) mindestens halb so lang wie die untere Wicklung (35) ist.

5. Sensor nach einem der Ansprüche 1 bis 4, wobei die obere Wicklung (33) einen größeren Abstand als die untere Wicklung (35) aufweist.

6. Sensor nach einem der Ansprüche 1 bis 5, wobei der dreidimensionale Resonanzwandler (31) ferner eine Metallabschirmung (71) um den HF Absorber (67) herum umfasst.

7. Sensorsystem (11), umfassend
einen Sensor nach einem der Ansprüche 1 bis 6;
eine Probenentnahme-Anordnung (13); und
einen Impedanz-Analysator (15); wobei der Impedanz-Analysator nur mit der oberen Wicklung (33) des Sensors verbunden ist.

8. Sensorsystem nach Anspruch 7, wobei:
der Resonanzwandler (31), einen Resonator umfasst, der konfiguriert ist, um einen Satz von LCR Resonanzschaltungsparametern zu messen; und
der Satz von LCR Resonanzschaltungsparametern mindestens eines von einem Impedanzspektrum, einem Realteil des Impedanzspektrums, einem Imaginärteil eines Implantatspektrums, sowohl Real-als auch Imaginärteile des Impedanzspektrums, einer Frequenz eines Maximums des Realteils einer komplexen Impedanz, einer Größe des Realteils der komplexen Impedanz, einer Resonanzfrequenz, einer Größe des Imaginärteils der komplexen Impedanz, und einer Anti-Resonanzfrequenz und einer Größe des Imaginärteils der komplexen Impedanz umfassen.

9. Sensorsystem nach Anspruch 8, wobei der Impedanz-Analysator (15) den Satz von LCR Resonanzschaltungsparametern in Werte eines komplexen Impedanzspektrums umwandelt.

10. Sensorsystem zum Bestimmen einer Zusammensetzung eines Gemischs von Öl und Wasser in einem Behälter, umfassend:
ein Untersystem, das einen Satz von komplexen Impedanzspektrumwerten des Öls an einem Ende des Behälters und des Wassers an dem gegenüberliegenden Ende mit einem Sensor gemäß einem der Ansprüche 1 bis 6 bestimmt;
ein Untersystem, das Kalibrierungswerte für das Sensorsystem für 100 % Öl bzw. 100 % Wasser erzeugt;
ein Untersystem, das ein Modell aus den Kalibrierungswerten erzeugt; und
ein Untersystem, das das Modell auf den Satz von komplexen Impedanzspektrumwerten anwendet, um die Zusammensetzung zu bestimmen.

11. Verfahren zum Messen einer Schnittstellenhöhe zwischen Fluids in einem Behälter, wobei das Verfahren Folgendes umfasst,
Erfassen eines Satzes von Signalen von einem Sensor gemäß einem der Ansprüche 1 bis 6 an einer Vielzahl von Orten in dem Behälter;
Umwandeln des Satzes von Signalen in Werte, die sich auf eine Impedanz des als Probe entnommenen Fluids für die Vielzahl von Orten beziehen; und
Bestimmen eines Fluidphasen-Inversionspunkts von den Werten.

12. Verfahren zum Bestimmen einer Zusammensetzung eines Gemischs von Partikeln in einer Flüssigkeit, umfassend:
Bestimmen, mit einem Sensorsystem (11) nach einem der Ansprüche 7-10, eines Werts, der sich auf die Impedanz des Gemischs bezieht; und
Anwenden eines Phasenmodells der Flüssigkeit auf den Wert.

## Revendications

1. Capteur comprenant:
un transducteur résonant tridimensionnel (31) configuré pour déterminer une composition d'une émulsion ou d'une autre dispersion,
dans lequel le transducteur résonant tridimensionnel comprend:
- une cellule d'échantillonnage (39);
- un enroulement supérieur (33) et un enroulement inférieur (35), l'enroulement inférieur (35) étant situé autour de la cellule d'échantillonnage (39) et l'enroulement supérieur (33) étant situé autour et concentrique à l'enroulement inférieur (35);
- un isolant galvanique prévu entre l'enroulement supérieur (33) et l'enroulement inférieur (35);
- une couche absorbante (67) de radiofréquence (RF) disposée autour de l'enroulement supérieur (33);
- un espaceur (72) situé entre l'enroulement supérieur (33) et la couche absorbante (67) de RF; l'espaceur (72) étant fait de matériau isolant galvanique;
- des câbles (74) connectant les extrémités de l'enroulement supérieur (33) à un connecteur (68), ledit connecteur étant adapté pour connecter un câble électrique d'un analyseur d'impédance (15) au transducteur résonant tridimensionnel (31);
- des raccords (34) aux extrémités de la cellule d'échantillonnage (39);
dans lequel:
l'enroulement supérieur (33) est adapté pour agir à la fois comme une bobine d'entraînement et une bobine de captage;
l'enroulement inférieur (35) est adapté pour agir comme un résonateur ou une bobine de détection,
l'enroulement inférieur (35) est un enroulement flottant, ne possédant pas de connexions galvaniques à d'autres parties du transducteur résonant tridimensionnel (31);
l'enroulement inférieur (35) est adapté pour être excité par un champ électromagnétique créé par une vague d'énergie circulant à travers l'enroulement supérieur (33) de manière que l'enroulement inférieur (35) excité génère un autre champ électromagnétique qui est altéré par son interaction avec le fluide dans la cellule d'échantillonnage (39), ledit champ électromagnétique altéré étant ensuite détecté par l'enroulement supérieur (33).

2. Capteur selon la revendication 1, dans lequel l'absorbeur RF (67) absorbe un rayonnement électromagnétique dans les plages de fréquences des kilohertz, mégahertz, gigahertz, et térahertz dépendamment de la fréquence de fonctionnement du transducteur résonant tridimensionnel (31) et de la source potentielle d'interférences.

3. Capteur selon la revendication 1 dans lequel l'absorbeur RF (67) est une combinaison de couches individuelles pour des plages de fréquences particulières.

4. Capteur selon l'une quelconque des revendications 1 à 3 dans lequel l'enroulement supérieur (33) est au moins la moitié de la longueur de l'enroulement inférieur (35).

5. Capteur selon l'une quelconque des revendications 1 à 4, dans lequel l'enroulement supérieur (33) a un pas supérieur à l'enroulement inférieur (35).

6. Capteur selon l'une quelconque des revendications 1 à 5, le transducteur résonant tridimensionnel (31) comprenant en outre un blindage métallique (71) autour de l'absorbeur RF (67).

7. Système capteur (11) comprenant
un capteur selon l'une quelconque des revendications 1 à 6;
un ensemble d'échantillonnage (13); et
un analyseur d'impédance (15); dans lequel l'analyseur d'impédance est connecté seulement à l'enroulement supérieur (33) du capteur.

8. Système capteur selon la revendication 7, dans lequel:
le transducteur résonant (31) comprend un résonateur configuré pour mesurer un ensemble de paramètres de circuit résonant LCR; et
l'ensemble de paramètres de circuit résonant LCR comprend au moins l'un parmi un spectre d'impédance, une partie réelle du spectre d'impédance, une partie imaginaire d'un spectre d'impédance, les deux des parties réelle et imaginaire du spectre d'impédance, une fréquence d'un maximum de la partie réelle d'une impédance complexe, une magnitude de la partie réelle de l'impédance complexe, une fréquence de résonance, une magnitude de la partie imaginaire de l'impédance complexe, et une fréquence anti-résonante et une magnitude de la partie imaginaire de l'impédance complexe.

9. Système capteur selon la revendication 8 dans lequel l'analyseur d'impédance (15) convertit l'ensemble de paramètres de circuit résonant LCR en valeurs d'un spectre d'impédance complexe.

10. Système capteur pour déterminer une composition d'un mélange d'huile et d'eau dans un récipient comprenant:
un sous-système qui détermine qu'un ensemble de valeurs de spectre d'impédance complexe de l'huile à une extrémité du récipient et de l'eau à l'extrémité opposée grâce à un capteur selon l'une quelconque des revendications 1 à 6;
un sous-système qui génère des valeurs de calibrage pour le système capteur pour 100 % d'huile et 100 % d'eau, respectivement;
un sous-système qui génère un modèle à partir des valeurs de calibration; et
un sous-système qui applique le modèle à l'ensemble de valeurs de spectre d'impédance complexe pour déterminer la composition.

11. Procédé pour mesurer une hauteur d'interface entre des fluides dans un récipient, le procédé comprenant,
la détection d'un ensemble de signaux d'un capteur selon l'une quelconque des revendications 1 à 6 au niveau d'une pluralité de localisations dans le récipient;
la conversion de l'ensemble de signaux en valeurs liées à une impédance du fluide échantillonné pour la pluralité de localisations; et,
la détermination d'un point d'inversion de phase du fluide à partir des valeurs.

12. Procédé pour déterminer une composition d'un mélange de particules dans un liquide comprenant
la détermination grâce à un système capteur (11) selon l'une quelconque des revendications 7 à 10 d'une valeur liée à l'impédance du mélange; et,
l'application d'un modèle de phase du liquide à la valeur.
